# DEMANDE DE BREVET EUROPEEN

(11) **EP 1 607 036 A1**
(43) Date de publication de la demande: **21.12.2005**
(21) Numéro de dépôt: 04447144.9
(22) Date de dépôt: 18.06.2004
(51) Int. Cl.: A61B 1/00, A61B 1/018

(54) **Support d' instruments comprenant une bague et montable sur un endoscope**

(71) Demandeur: UNIVERSITE LIBRE DE BRUXELLES, 1050 Bruxelles (BE)
(72) Inventeur: Deviere, Jacques, B-1470 Genappe (BE); Cauche, Nicolas, B-1040 Bruxelles (BE); Delchambre, Alain, B-1170 Bruxelles (BE); Evrard, Sylvie, B-1190 Bruxelles (BE)
(74) Mandataire: Van Malderen, Joelle

(57) **Abrégé**

La présente invention se rapporte à une table endoscopique (1) comprenant une bague d'assemblage (2) à un endoscope et au moins deux bras opérateurs (3,3') solidarisés à ladite bague d'assemblage (2) et aptes à être traversés chacun par au moins un instrument chirurgical, lesdits bras opérateurs (3,3') présentant chacun au moins un premier degré de liberté en rotation suivant lequel chacun des bras opérateurs (3,3') est orientable indépendamment de l'autre.

La présente invention concerne également des kits endoscopiques comprenant ladite table.

## Description

### Objet de l'invention

La présente invention se rapporte à un instrument utilisable en endoscopie.

La présente invention concerne plus particulièrement un support endoscopique encore appelé ci-après table endoscopique et destiné à permettre le positionnement *in situ* d'un endoscope et d'instruments chirurgicaux associés de façon précise.

### Etat de la technique

L'endoscopie thérapeutique s'est considérablement développée au cours de ces vingt dernières années. Pratiquée par voie perorale ou transanale, l'endoscopie thérapeutique permet, au moyen d'un instrument appelé « endoscope », un accès direct à l'intérieur de l'oesophage ou de l'estomac sans nécessiter l'ouverture de la paroi abdominale ni le passage par la cavité péritonéale, ce qui augmente le confort et diminue la morbidité pour le patient.

Plus précisément, un endoscope se présente classiquement sous la forme d'un tube souple présentant un canal optique et un ou plusieurs canaux opérateurs permettant d'introduire un ou plusieurs instruments chirurgicaux en vue de pratiquer une manipulation chirurgicale *in situ.*

Cependant, une telle disposition des canaux opérateurs, parallèlement à l'endoscope, limite considérablement les mouvements dans l'espace accessibles aux instruments chirurgicaux l'un par rapport à l'autre et par rapport à l'endoscope, et il conviendrait de disposer d'une solution efficace à ce problème.

Le document US-B1-6,352,503 a proposé un appareil d'endoscopie chirurgicale comprenant un endoscope pourvu d'une zone d'insertion à l'intérieur de laquelle est inséré un dispositif d'observation, et au moins deux bras d'insertion dans lesquels un instrument chirurgical est inséré, ledit appareil ayant la particularité de comprendre en outre un dispositif de positionnement, pour faire varier l'une ou l'autre des distances entre la zone d'insertion de l'endoscope, le premier bras d'insertion et le second bras d'insertion selon une direction essentiellement orthogonale à l'axe de la zone d'insertion de l'endoscope. Ce dispositif de positionnement se présente de préférence sous la forme d'un ballon gonflable ou également d'un « panier » extensible.

Néanmoins, dans cette solution, les mouvements dans l'espace des instruments chirurgicaux sont encore relativement restreints et l'orientation des bras d'insertion ne sont pas indépendants l'un de l'autre.

### Buts de l'invention

La présente invention vise à fournir un instrument comprenant un dispositif de positionnement ou d'orientation d'un endoscope et d'instruments chirurgicaux couplés audit endoscope qui permettrait de positionner ou d'orienter précisément dans l'espace lesdits instruments chirurgicaux l'un par rapport à l'autre et par rapport audit endoscope.

En particulier, la présente invention vise à fournir un instrument compatible avec une utilisation *in situ* dans la lumière du tube digestif.

Un autre but de la présente invention est de fournir un instrument adaptable à une large gamme d'instruments chirurgicaux et d'endoscopes.

La présente invention vise également à fournir un instrument qui soit facile d'utilisation et d'entretien, tout en garantissant une sécurité maximale.

Enfin, un autre but de l'invention est de fournir un instrument qui permette des manipulations chirurgicales par voie endoluminale, augmentant le confort pour le patient.

### Résumé de l'invention

La présente invention se rapporte à une table ou support endoscopique comprenant une bague d'assemblage à un endoscope et au moins deux bras opérateurs solidarisés à ladite bague d'assemblage et aptes à être traversés chacun par au moins un instrument chirurgical, lesdits bras opérateurs présentant chacun au moins un premier degré de liberté en rotation suivant lequel chacun des bras opérateurs est orientable indépendamment de l'autre.

De préférence, chaque bras opérateur est orientable selon le premier degré de liberté en rotation d'un premier angle variable compris dans un premier plan d'orientation.

Avantageusement, chacun desdits bras opérateurs présente un deuxième degré de liberté en rotation suivant lequel chacun des bras opérateurs est orientable indépendamment de l'autre.

De préférence, chaque bras opérateur est orientable selon le deuxième degré de liberté en rotation d'un deuxième angle variable compris dans un deuxième plan d'orientation.

De préférence, la table endoscopique selon l'invention comprend un dispositif d'orientation apte à orienter les bras opérateurs selon leur(s) degré(s) de liberté.

De préférence, la table endoscopique présente au moins deux positions, une position de repli correspondant à un volume minimal de ladite table et une position de déploiement correspondant à une position d'assemblage à l'endoscope.

De préférence, la table endoscopique comprend un dispositif de repli/déploiement apte à faire passer la table d'une position à l'autre.

De préférence, la table endoscopique est apte à être couplée à des moyens dits d'activation ou de contrôle manuels ou automatiques pour commander l'actionnement du dispositif d'orientation et/ou l'actionnement du dispositif de repli/déploiement.

Selon une première forme préférée d'exécution de l'invention, la bague d'assemblage de la table endoscopique comprend une membrane flexible (5) et un câble dit câble d'assemblage, ladite bague étant repliée en position de repli de la table et déployée en position d'assemblage de la table.

De préférence, le dispositif de repli/déploiement de la table comprend ledit câble d'assemblage et un câble dit de repli et ledit dispositif est actionnable par l'intermédiaire de guides métalliques.

Selon une seconde forme préférée d'exécution de l'invention, la bague d'assemblage à l'endoscope se présente sous la forme d'une structure rigide avec un axe principal.

De préférence, le dispositif de repli/déploiement de la table correspond à un dispositif de pivotement qui est apte à faire pivoter ladite bague autour de son axe principal d'un certain angle.

Avantageusement dans la présente invention, en position de repos de la table, les deux bras opérateurs sont orientés essentiellement parallèlement l'un par rapport à l'autre et essentiellement perpendiculairement à la bague d'assemblage.

De préférence, les deux bras opérateurs sont disposés en vis-à-vis l'un de l'autre par rapport au diamètre de la table.

De préférence selon l'invention, le dispositif d'orientation des bras opérateurs comprend un ensemble de câbles situés au niveau desdits bras opérateurs et actionnables en mouvement.

De préférence selon l'invention, le premier plan d'orientation des bras opérateurs est défini par les deux axes principaux desdits bras opérateurs.

De préférence selon l'invention, le deuxième plan d'orientation des bras opérateurs est défini par l'axe principal dudit bras opérateur et un deuxième axe correspondant à l'axe essentiellement perpendiculaire au premier plan d'orientation.

Avantageusement, les bras opérateurs sont pourvus de marqueurs optiques.

Selon une troisième forme préférée d'exécution de l'invention, la bague d'assemblage à l'endoscope se présente sous la forme d'une membrane gonflable, la bague étant non gonflée en position de repli de la table et gonflée en position d'assemblage de la table.

De préférence, le dispositif d'orientation se présente sous la forme d'un circuit de circulation de fluide comprenant au moins des compartiments situés au niveau des bras opérateurs et susceptibles d'être remplis par un fluide selon un taux de remplissage ajustable.

La présente invention se rapporte également à un kit endoscopique comprenant une table endoscopique selon l'invention et des moyens d'activation automatique ou des moyens d'activation manuelle du dispositif d'orientation des bras opérateurs et/ou de déploiement/repliement de la table.

La présente invention se rapporte également à un kit endoscopique comprenant une table endoscopique selon l'invention et un endoscope et/ou au moins un instrument chirurgical.

L'invention concerne aussi un kit endoscopique, comprenant en outre et des moyens d'activation automatique ou des moyens d'activation manuelle du dispositif d'orientation des bras opérateurs et/ou de déploiement/repliement de la table.

### Définitions :

On notera que dans la présente invention les termes « positionnement » et « orientation » sont synonymes.

On doit donc comprendre que dans la présente invention une position ou une orientation des bras opérateurs correspond à un jeu précis de coordonnées dans l'espace.

Ainsi que l'on pourra le comprendre aussi dans la présente description, la table endoscopique selon l'invention permet de positionner un endoscope et des instruments chirurgicaux à une certaine hauteur dans le tube digestif d'un patient mais également une fois cette hauteur réglée d'affiner précisément la position des bras opérateurs et des instruments chirurgicaux.

La notion de « degré de liberté » d'un système telle qu'utilisée dans la présente invention correspond à celle communément utilisée par l'homme de l'art en mécanique et désigne a priori aussi bien un degré de liberté en rotation qu'un degré de liberté en translation.

La notion de « position d'assemblage » de la table renvoie à une conformation particulière de la table dans laquelle elle peut être assemblée à un endoscope. Cette position d'assemblage de la table correspond elle-même à une certaine conformation de la bague d'assemblage de la table.

### Brève description des figures

Les figures 1a et 1b représentent une vue générale d'un support ou d'une table endoscopique selon une première forme préférée d'exécution de la présente invention.

Les figures 2a-2b correspondent à deux vues différentes de cette même table endoscopique, avec un endoscope assemblé sur cette table.

Les figures 3a et 3b correspondent à deux vues différentes montrant en détail la bague d'assemblage à l'endoscope dans cette première forme préférée d'exécution d'une table selon l'invention.

La figure 4 représente une table selon une seconde forme préférée d'exécution de l'invention, en position repliée (vue de face à gauche ; vue de dessus à droite).

La figure 5 représente cette même table mais en position dépliée (vue de face à gauche ; vue de dessus à droite).

La figure 6 montre comment un endoscope et des instruments chirurgicaux peuvent être assemblés sur cette table.

La figure 7 représente une table endoscopique selon une troisième forme préférée d'exécution de la présente invention.

### Description détaillée de l'invention

### Première forme préférée d'exécution :

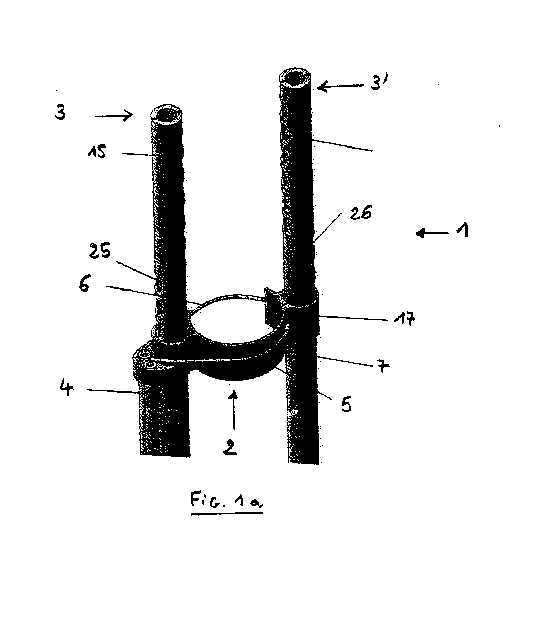

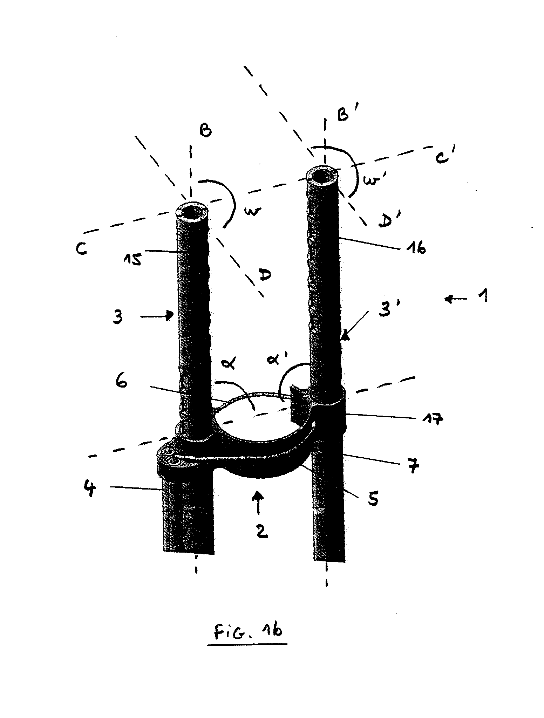

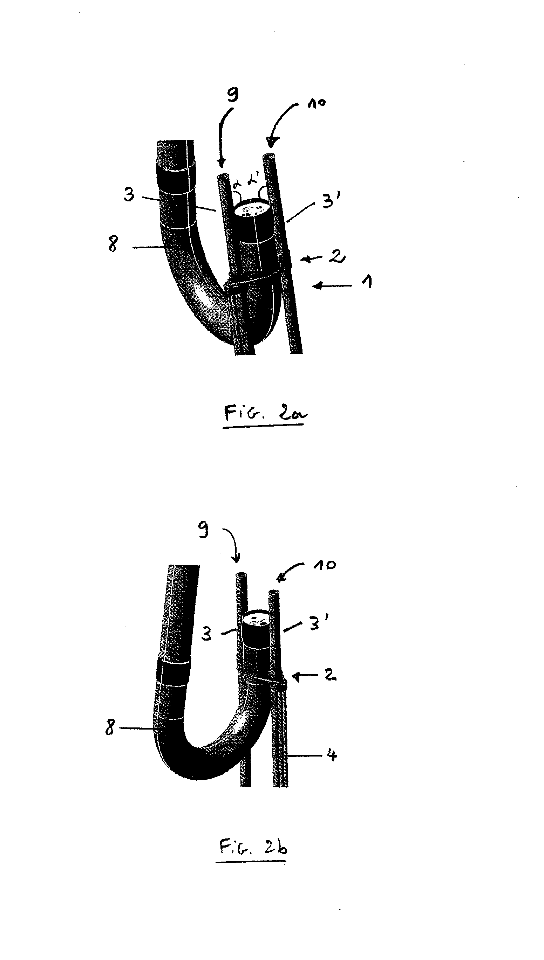

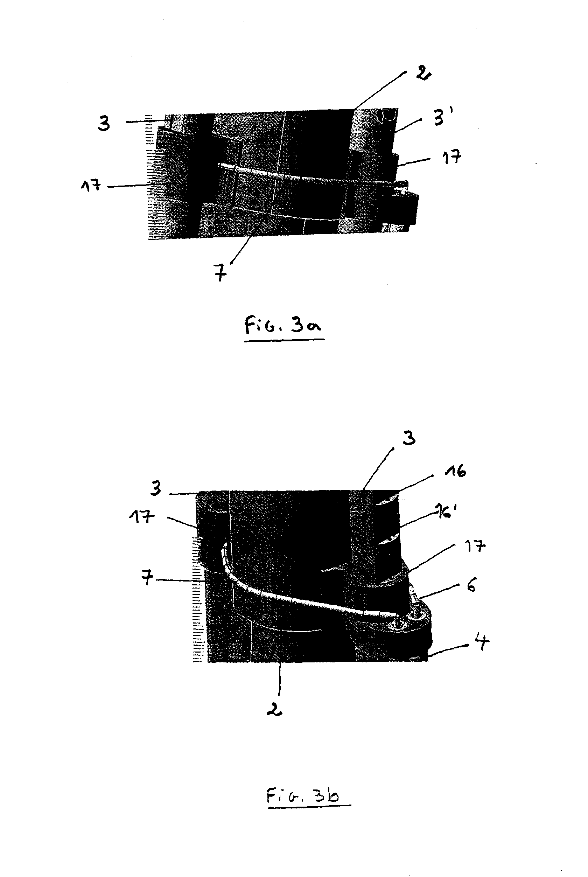

Un exemple d'une première forme préférée de la table opératoire selon l'invention est présenté aux figures 1a-1b, 2a-2b, 3a-3b.

La table endoscopique 1 comprend une bague d'assemblage ou interface d'assemblage 2 adaptée pour l'assemblage à un endoscope, et deux bras opérateurs 3,3' solidarisés à ladite bague d'assemblage 2 par des moyens de solidarisation 17, de préférence rigides.

Les bras opérateurs 3,3' se présentent sous la forme de tubes flexibles et comportent au moins un lumen ou canal configuré de façon telle que l'on peut y faire passer des outils ou instruments chirurgicaux de différentes formes.

La table endoscopique 1 présente au moins deux positions, une position de repli correspondant à un volume minimal de ladite table tel que ladite table puisse être introduite dans le corps d'un patient par les voies naturelles (dites endoluminales c'est-à-dire perorales ou transanales) et une position de déploiement correspondant à une position d'assemblage à l'endoscope, c'est-à-dire une position dans laquelle un endoscope peut être assemblé à la table. Cette position est celle que la table peut adopter une fois qu'elle a atteint sa cible, par exemple l'oesophage ou l'estomac.

Un dispositif de repli/déploiement de la table 1 est prévu à cet effet (voir ci-après).

En position d'assemblage de la table, la bague d'assemblage 2 est déployée, tandis qu'en position de repli de la table cette bague 2 est repliée sur elle-même.

Dans cette première forme préférée d'exécution, la bague d'assemblage 2 comprend une membrane flexible 5 qui contribue avec au moins un câble dit « câble d'assemblage » 6 à la forme de la bague 2 en position d'assemblage de la table 1.

La bague d'assemblage 2 comprend également au moins un câble dit « câble de repli » 7 qui permet de replier la bague 2 lorsque la table 1 passe de la position d'assemblage à la position de repli.

Le câble de repli 7 et le câble d'assemblage 6 forment ensemble le dispositif de repli/déploiement de la bague 2 et donc de la table 1. Ce dispositif (6,7) est actionnable par l'intermédiaire de guides métalliques 4 disposés de préférence au niveau des bras opérateurs 3 et 3', mais d'autres moyens équivalents peuvent également être prévus à cet effet.

L'actionnement en mouvement (la mise en mouvement) du câble de repli 7 et du câble d'assemblage 6 du dispositif de repli/déploiement est commandé soit manuellement soit automatiquement avec des moyens de contrôle par l'intermédiaire desdits guides métalliques 4.

En position de repos de la table 1, les deux bras 3 et 3' sont orientés essentiellement parallèlement l'un par rapport à l'autre et essentiellement perpendiculairement à la bague d'assemblage 2.

En outre, les deux bras 3 et 3' sont disposés en vis-à-vis l'un de l'autre par rapport au diamètre de la bague 2.

Avantageusement, les bras opérateurs 3 et 3' sont pourvus de marqueurs optiques (non représentés).

Selon un aspect essentiel de l'invention, chacun des bras opérateurs 3 et 3' présente au moins un degré de liberté qui correspond à une rotation dans un premier plan d'orientation défini par les deux axes principaux B et B' desdits bras opérateurs 3 et 3' respectivement. Chacun des bras opérateurs 3 et 3' est orientable (inclinable) dans ce premier plan d'un certain angle α ou α' de façon indépendante grâce à un dispositif d'orientation.

En d'autres termes, les bras 3 et 3' sont orientables indépendamment l'un de l'autre dans au moins deux dimensions de l'espace, grâce à ce dispositif d'orientation.

Dans cette première forme préférée d'exécution, le dispositif d'orientation des bras opérateurs 3,3' (encore appelé dispositif de positionnement) comprend un ensemble de câbles 15, 25 d'une part et 16, 26, d'autre part, ces câbles étant situés au niveau respectivement du bras opérateur 3 et du bras opérateur 3' (deux séries de câbles sur chacun des bras opérateurs 3 et 3' assurant des déplacements en sens opposé). Ces câbles sont actionnables en mouvement par des moyens de contrôle mécaniques ou automatiques, encore appelés moyens d'activation ou moyens d'actionnement.

De façon générale, l'orientation des bras opérateurs 3 et 3' est ajustée par le dispositif d'orientation de manière à ce que lorsque la table endoscopique est assemblée à l'endoscope, les bras opérateurs ne gênent pas la visibilité conférée par l'endoscope.

Il est à noter que le dispositif d'orientation est conçu pour orienter les bras 3 et 3' avec des valeurs d'angles α, α' telles qu'en conditions d'utilisation, quand les instruments chirurgicaux 9,10 sont fixés auxdits bras 3 et 3', lesdits instruments chirurgicaux 9,10 se fassent toujours face.

De façon particulièrement avantageuse, chacun des bras opérateurs 3 et 3' présente en outre un autre degré de liberté (deuxième degré de liberté) qui correspond à une rotation dans un deuxième plan d'orientation défini d'une part par l'axe principal B ou B' dudit bras opérateur 3 ou 3' respectivement et d'autre part par un deuxième axe D ou D' respectivement correspondant à un axe perpendiculaire au premier plan d'orientation défini ci-dessus. (On notera comme représenté sur la fig.1b que CBD et C'B'D' forment chacun deux trièdres rectangles). Chacun des bras opérateurs 3 et 3' est orientable (inclinable) dans ce deuxième plan d'orientation d'un certain angle ω ou ω' respectivement de façon indépendante grâce au dispositif d'orientation.

A cet effet, le dispositif d'orientation des bras opérateurs 3,3' comprend des câbles supplémentaires 35,45,... et 36,46,... (non représentés sur les figures) situés au niveau des bras opérateurs respectivement 3 et 3' et actionnables en mouvement par des moyens de contrôle mécaniques ou automatiques de façon à orienter les bras 3 et 3' spécifiquement dans le deuxième plan d'orientation défini par ces angles ω et ω'.

En d'autres termes, avantageusement, les bras 3 et 3' sont orientables indépendamment l'un de l'autre dans les trois dimensions de l'espace, grâce au dispositif d'orientation.

D'autres câbles peuvent encore être ajoutés de façon à accroître, de manière indépendante, la mobilité des instruments chirurgicaux 9 et 10.

On peut également prévoir d'autres degrés de liberté pour les bras opérateurs 3 et 3' qui permettraient de les orienter encore plus précisément dans l'espace. Ainsi, on peut prévoir que les bras 3 et 3' soient également orientables en translation le long de leurs axes principaux et de façon indépendante l'un de l'autre et le dispositif d'orientation peut être adapté à cet effet.

On peut également prévoir de pouvoir faire tourner les bras opérateurs sur eux-mêmes (autour de leurs axes principaux respectifs).

En pratique, lorsqu'il s'agit d'utiliser la table opératoire selon l'invention, le chirurgien peut procéder de la façon suivante.

La table opératoire 1 en position repliée est introduite par voie endoluminale, par exemple perorale, dans le tube digestif du patient. Une fois que la table opératoire a atteint la cible à traiter, par exemple l'estomac ou l'oesophage, le chirurgien commande par voie manuelle ou automatique via l'actionnement du dispositif de repli/déploiement le déploiement de la table 1 : la bague d'assemblage 2 qui était repliée se déploie alors. Plus précisément, le câble d'assemblage 6 est actionné et forme avec la membrane flexible 5 la bague d'assemblage 2 avec son orifice ou trou 11. La table 1 est alors dans sa position de repos ou d'équilibre.

L'endoscope 8 est alors inséré dans l'orifice 11 de la bague d'assemblage 2 et des instruments chirurgicaux 9 et 10 sont insérés au niveau des bras opérateurs 3 et 3' respectivement.

Le dispositif d'orientation est alors à son tour actionné par les moyens de contrôle manuel ou automatique de manière à mettre en mouvement dans au moins deux directions de l'espace (angles α et α' respectivement) les bras opérateurs 3 et 3', voire dans les trois directions de l'espace (angles α et α' respectivement d'une part, et ω et ω' respectivement d'autre part, avec éventuellement une translation des bras 3 et 3'). Le résultat est le positionnement ainsi de façon précise des instruments chirurgicaux 9 et 10 respectivement par rapport à la cible à traiter et par rapport à l'endoscope 8.

La table endoscopique peut en outre être conçue de manière à permettre une orientation des instruments chirurgicaux 9 et 10 en translation dans les bras opérateurs 3 et 3' et en rotation autour de leurs axes, sous l'actionnement des moyens de contrôle.

Une fois les instruments ainsi correctement positionnés, la procédure chirurgicale opératoire peut commencer. Au cours de la procédure, les moyens de contrôle peuvent actionner le dispositif d'orientation en vue d'ajuster à tout moment la position en 3D des bras opérateurs 3,3' et donc des instruments chirurgicaux 9,10.

Un système de sécurité incluant des moyens d'alarme peut en outre être prévu de manière à pouvoir à tout moment en cas de problème interrompre la procédure et désassembler si besoin rapidement la table opératoire 1, l'endoscope et les instruments chirurgicaux.

Une fois la procédure chirurgicale terminée, l'endoscope 8 et les instruments chirurgicaux 9,10 sont ôtés de la table opératoire 1 et le dispositif de repli/déploiement est à nouveau actionner pour replier ladite table 1. La table 1 ainsi repliée peut alors être retirée du corps du patient.

Il est à noter que la composition et les dimensions de la table endoscopique 1 sont telles qu'elles sont compatibles avec son utilisation. En d'autres termes on pourra sélectionner les matériaux pour fabriquer les différents éléments de la table parmi les matériaux biocompatibles tels que le silicone, les polymères PTFE, PVC, PP, PS, PET, un acier inoxydable, le nitinol ou le titane.

On choisira aussi la taille de la bague d'assemblage 2 (épaisseur, hauteur h et périmètre) de façon à optimiser la surface de contact avec l'endoscope et obtenir ainsi notamment un assemblage stable et sécurisé.

De même, en ce qui concerne la fabrication de la table endoscopique selon l'invention, différents procédés peuvent être envisagés pour fabriquer les différents éléments tels que l'injection, l'extrusion, le marquage au laser ou l'usinage.

En outre, l'assemblage de ces différents éléments pourra être réalisé selon différentes techniques telles que le collage, le soudage tel que le soudage ultrason, ou l'injection de bi-composants.

Par ailleurs, il est à noter que si dans l'exemple décrit ci-dessus le dispositif de repli/déploiement de la bague d'assemblage 2 et donc de la table 1 comprend un ensemble de câbles reliés à des guides métalliques, d'autres dispositifs alternatifs ayant la même fonction peuvent aussi être envisagés par exemple un dispositif à glissière interne, à glissière externe, un dispositif d'enroulement autour d'un bras opérateur.

On notera que dans cette forme d'exécution, la membrane flexible 5 peut être remplacée par tout moyen équivalent tel que par exemple un ou plusieurs câbles.

On pourrait également envisager que la membrane 5 ne soit plus flexible mais rigide.

### Description d'une seconde forme préférée d'exécution :

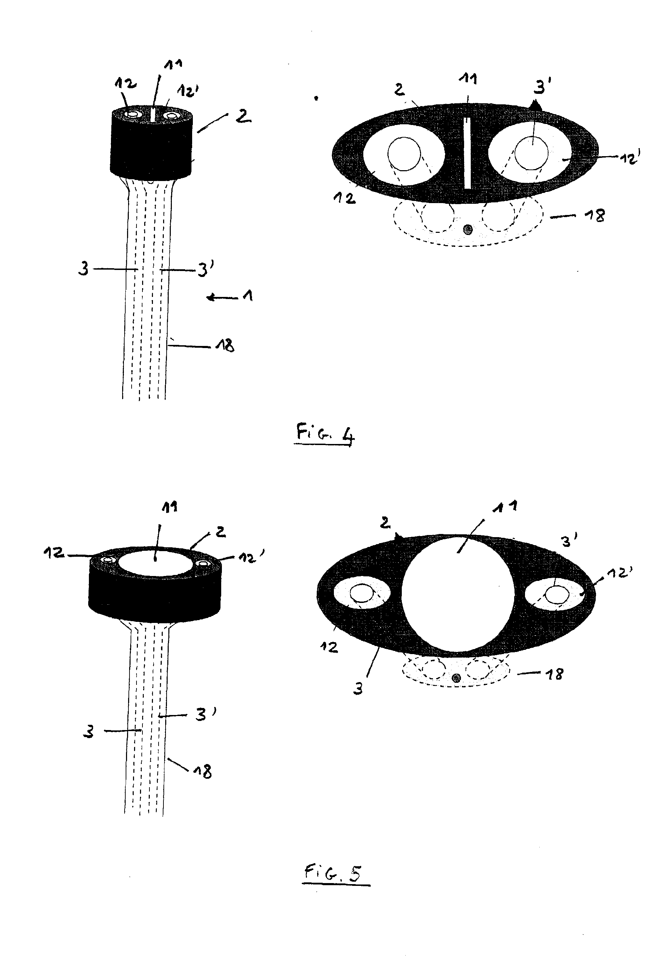

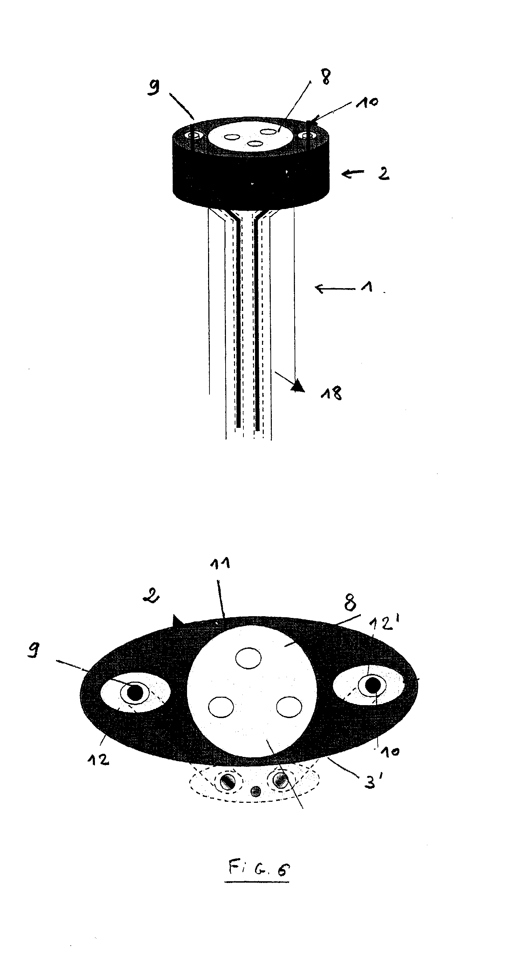

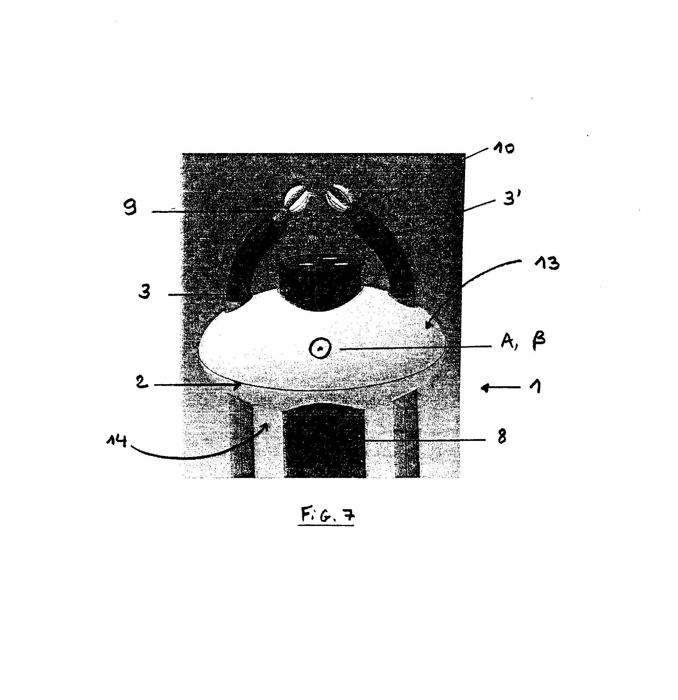

Une seconde forme préférée d'exécution de la table endoscopique selon l'invention est illustrée aux figures 4 à 6.

Dans cette seconde forme d'exécution, la bague ou interface d'assemblage 2 prend la forme d'une structure ou membrane gonflable pourvue en son centre d'un orifice dit « orifice endoscopique » 11 dont la taille varie en fonction de l'état de gonflement de la membrane 2. Lorsque la membrane 2 est suffisamment gonflée la taille de l'orifice endoscopique 11 est telle qu'un endoscope 8 peut y être fixé (par exemple par emboîtement).

La membrane 2 comporte aussi dans son épaisseur des passages 12,12' pour les bras opérateurs 3,3' qui la traversent de part en part, tandis que la partie des bras opérateurs 3,3' située en dehors de la membrane 2 est maintenue de préférence dans un guide endoscopique 18 qui la protège vis-à-vis de l'environnement.

Différents moyens de gonflement de la membrane 2 peuvent être envisagés. Par exemple, le gonflement peut être obtenu par la circulation d'un fluide (liquide ou gaz) sous pression à l'intérieur d'un canal ou d'un compartiment d'amenée de fluide.

De même, différents dispositifs d'orientation des bras opérateurs 3,3' peuvent être envisagés. Par exemple, la membrane peut être pourvue de un ou plusieurs canaux ou compartiments aptes à recevoir un fluide (liquide ou gaz) sous pression et agencés au sein de la membrane 2 de façon à exercer une force plus ou moins grande sur les bras opérateurs 3,3' et ainsi ajuster leur positionnement en trois dimensions, et donc celui des instruments chirurgicaux 9,10.

Dans ces cas, la table 1 et plus particulièrement la bague ou interface d'assemblage 2 doit donc être reliée à une source externe de fluide.

### Troisième forme préférée d'exécution :

Une troisième forme préférée d'exécution de la table endoscopique selon l'invention est représentée à la Fig.7.

Dans cette troisième forme d'exécution, la bague ou interface d'assemblage 2 prend la forme d'une structure rigide capable de pivoter autour de l'axe A, de préférence d'un angle de pivotement ou de rotation β d'environ 90° de manière à être repliée ou au contraire déployée. En position déployée, la bague ou interface d'assemblage 2 a son axe principal essentiellement perpendiculaire aux bras opérateurs 3,3'. Si on définit dans cette position une face nord 13 et une face sud 14 pour la bague 2, la position repliée de la bague 2 correspond alors à une position de la bague dans laquelle la face nord 13 et la face sud 14 sont cette fois-ci situées dans des plans parallèles aux bras opérateurs 3,3'.

En d'autres termes, en pratique lorsqu'il s'agit d'introduire la table endoscopique 1 dans le corps d'un patient, par exemple dans l'oesophage, la table 1 est repliée et donc la bague ou interface d'assemblage 2 expose sa face nord 13 et sa face sud 14 dans l'axe de l'oesophage.

Un dispositif de repli/déploiement de la bague 2 et donc de la table endoscopique 1 à l'aide d'un câble relié à des guides métalliques peut être prévu pour mettre en oeuvre un tel changement de conformation.

L'actionnement de ce dispositif de repli/déploiement peut se faire, comme pour la première forme d'exécution par des moyens de commande ou contrôle manuels ou automatiques.

D'autres alternatives d'exécution de la table 1 peuvent encore être prévues telles que par exemple une table avec une demi-bague.

### Avantages de la table endoscopique selon l'invention :

Ainsi qu'illustré ci-dessus, grâce au dispositif d'orientation des bras opérateurs 3,3', il est possible avec la table endoscopique selon l'invention d'obtenir un positionnement précis des outils chirurgicaux dans le champ de vision de l'endoscope et selon un angle d'inclinaison variable de sorte que l'abord chirurgical s'en trouve facilité.

En outre, dans la mesure où les bras opérateurs 3,3' sont relativement flexibles, la table endoscopique selon l'invention peut être utilisée avec des outils chirurgicaux de formes très variées, y compris des outils chirurgicaux anguleux (non droits).

Un autre avantage de la table endoscopique selon l'invention est qu'elle n'est solidarisée ni aux outils chirurgicaux ni à l'endoscope. De par sa conception elle est donc capable de s'adapter à toutes sortes d'endoscopes et d'outils chirurgicaux.

De ce fait, même si la table endoscopique est avantageusement conçue pour être à usage unique, dans le cas où la table est conçue pour être réutilisée, son entretien s'en trouve également facilité tant au niveau simplicité qu'au niveau du coût et au niveau du temps.

Les applications médicales potentielles de l'instrument selon l'invention sont nombreuses et concernent notamment le traitement de pathologies telles que le reflux gastro-oesophagien, l'obésité morbide, la résection de tumeurs gastriques, la création d'anastomoses entre diverses structures digestives, et bien d'autres encore.

En conclusion, l'instrument selon la présente invention doit donc être compris comme un outil technique à la disposition du chirurgien pour faciliter la réalisation d'actes chirurgicaux tels que des sutures, des sections précises et des résections dans le cadre de l'endoscopie thérapeutique interventionnelle.

## Revendications

1. Table endoscopique (1) comprenant une bague d'assemblage (2) à un endoscope et au moins deux bras opérateurs (3,3') solidarisés à ladite bague d'assemblage (2) et aptes à être traversés chacun par au moins un instrument chirurgical, lesdits bras opérateurs (3,3') présentant chacun au moins un premier degré de liberté en rotation suivant lequel chacun des bras opérateurs (3,3') est orientable indépendamment de l'autre.

2. Table endoscopique selon la revendication 1, **caractérisée en ce que** chaque bras opérateur (3 ou 3') est orientable selon le premier degré de liberté en rotation d'un premier angle variable (α ou α') compris dans un premier plan d'orientation.

3. Table endoscopique selon la revendication 1 ou 2, **caractérisée en ce que** chacun desdits bras opérateurs (3,3') présente un deuxième degré de liberté en rotation suivant lequel chacun des bras opérateurs (3,3') est orientable indépendamment de l'autre.

4. Table endoscopique selon la revendication 1, **caractérisée en ce que** chaque bras opérateur (3 ou 3') est orientable selon le deuxième degré de liberté en rotation d'un deuxième angle variable (ω ou ω') compris dans un deuxième plan d'orientation.

5. Table endoscopique selon l'une des revendications précédentes, **caractérisée en ce qu'**elle comprend un dispositif d'orientation apte à orienter les bras opérateurs (3,3') selon leur(s) degré(s) de liberté.

6. Table endoscopique selon l'une des revendications précédentes, **caractérisée en ce qu'**elle présente au moins deux positions, une position de repli correspondant à un volume minimal de ladite table et une position de déploiement correspondant à une position d'assemblage à l'endoscope.

7. Table endoscopique selon la revendication 6, **caractérisée en ce qu'**elle comprend un dispositif de repli/déploiement apte à faire passer la table d'une position à l'autre.

8. Table endoscopique selon la revendication 5 à 7, **caractérisée en ce qu'**elle est apte à être couplée à des moyens dits d'activation ou de contrôle manuels ou automatiques pour commander l'actionnement du dispositif d'orientation et/ou l'actionnement du dispositif de repli/déploiement.

9. Table endoscopique selon l'une des revendications précédentes, **caractérisée en ce que** la bague d'assemblage (2) comprend une membrane flexible (5) et un câble dit câble d'assemblage (6), ladite bague étant repliée en position de repli de la table et déployée en position d'assemblage de la table.

10. Table endoscopique selon la revendication 9, **caractérisée en ce que** le dispositif de repli/déploiement de la table comprend ledit câble d'assemblage (6) et un câble dit de repli (7) et ledit dispositif est actionnable par l'intermédiaire de guides métalliques (4).

11. Table endoscopique selon l'une des revendications 1 à 8, **caractérisée en ce que** la bague d'assemblage à l'endoscope se présente sous la forme d'une structure rigide avec un axe principal (A).

12. Table endoscopique selon la revendication 12, **caractérisée en ce que** le dispositif de repli/déploiement de la table correspond à un dispositif de pivotement qui est apte à faire pivoter ladite bague (2) autour de son axe principal (A) d'un certain angle (β).

13. Table endoscopique selon l'une des revendications précédentes, **caractérisée en ce qu'**en position de repos de la table (1), les deux bras opérateurs (3,3') sont orientés essentiellement parallèlement l'un par rapport à l'autre et essentiellement perpendiculairement à la bague d'assemblage (2).

14. Table endoscopique selon l'une des revendications précédentes, **caractérisée en ce que** les deux bras opérateurs (3,3') sont disposés en vis-à-vis l'un de l'autre par rapport au diamètre de la table (1).

15. Table endoscopique selon l'une des revendications précédentes, **caractérisée en ce que** le dispositif d'orientation comprend un ensemble de câbles (15,25; 16,26, et éventuellement 35,45; 36,46) situés au niveau des bras opérateurs (3,3') et actionnables en mouvement.

16. Table endoscopique selon l'une des revendications précédentes, **caractérisée en ce que** le premier plan d'orientation des bras opérateurs (3,3') est défini par les deux axes principaux (B et B') desdits bras opérateurs (3 et 3').

17. Table endoscopique selon la revendication 16, **caractérisée en ce que** le deuxième plan d'orientation des bras opérateurs (3,3') est défini par l'axe principal (B ou B') dudit bras opérateur (3 ou 3') et un deuxième axe (D ou D') correspondant à l'axe essentiellement perpendiculaire au premier plan d'orientation.

18. Table endoscopique selon l'une des revendications précédentes, **caractérisée en ce que** les bras opérateurs (3,3') sont pourvus de marqueurs optiques.

19. Table endoscopique selon l'une des revendications 1 à 8, **caractérisée en ce que** la bague d'assemblage (2) à l'endoscope se présente sous la forme d'une membrane gonflable, la bague (2) étant non gonflée en position de repli de la table (1) et gonflée en position d'assemblage de la table (1).

20. Table endoscopique selon la revendication 19 **caractérisée en que** le dispositif d'orientation se présente sous la forme d'un circuit de circulation de fluide comprenant au moins des compartiments situés au niveau des bras opérateurs (3,3') et susceptibles d'être remplis par un fluide selon un taux de remplissage ajustable.

21. Kit endoscopique comprenant une table endoscopique selon l'une des revendications précédentes et des moyens d'activation automatique ou des moyens d'activation manuelle du dispositif d'orientation des bras opérateurs (3,3') et/ou de déploiement/repliement de la table (1).

22. Kit endoscopique comprenant une table endoscopique selon l'une des revendications précédentes et un endoscope (8) et/ou au moins un instrument chirurgical (9 ou 10).

23. Kit endoscopique selon la revendication 22, comprenant en outre et des moyens d'activation automatique ou des moyens d'activation manuelle du dispositif d'orientation des bras opérateurs (3,3') et/ou de déploiement/repliement de la table (1).
